# EUROPEAN PATENT SPECIFICATION

(11) **EP 2 149 362 B1**
(45) Date of publication and mention of the grant of the patent: **02.11.2016**
(21) Application number: 08752823.8
(22) Date of filing: 15.05.2008
(51) Int. Cl.: A61K 8/02, A61K 8/14, A61K 8/06, A61K 8/34, A61Q 5/12, A61Q 13/00, A61Q 19/00, A61K 8/891, A61K 8/894

(54) **VESICLE-CONTAINING COMPOSITION, AND METHOD FOR PRODUCTION THEREOF**
VESIKELHALTIGE ZUSAMMENSETZUNG UND HERSTELLUNGSVERFAHREN DAFÜR
COMPOSITION CONTENANT DES VÉSICULES, ET SON PROCÉDÉ DE FABRICATION

(30) Priority: 15.05.2007 JP 2007129777
(43) Date of publication of application: 03.02.2010
(73) Proprietor: Shiseido Company, Ltd., Chuo-ku Tokyo 104-8010 (JP)
(72) Inventor: WATANABE, Kei, Yokohama-shi Kanagawa 224-8558 (JP); ARAKI, Hidefumi, Yokohama-shi Kanagawa 224-8558 (JP); NAGARE, Yuko, Yokohama-shi Kanagawa 224-8558 (JP); NAKAMA, Yasunari, Yokohama-shi Kanagawa 224-8558 (JP); ISHINO, Hirokazu, Yokohama-shi Kanagawa 224-8558 (JP)
(74) Representative: Lippert, Stachow & Partner
(86) International application number: PCT/JP2008/058965
(87) International publication number: WO 2008/143140

(56) References cited:
- EP-A1- 2 196 185
- WO-A1-2005/103118
- WO-A1-2006/091295
- WO-A2-2005/102248
- WO-A2-2005/102248
- JP-A- 07 323 222
- JP-A- 08 239 475
- JP-A- 09 175 930
- JP-A- 09 175 931
- JP-A- 2006 513 280
- JP-A- 2008 024 681

## Description

### [FIELD OF THE INVENTION]

The present invention relates to a vesicle-containing composition and the production method thereof, and in particular, relates to the incorporating a perfume component into the vesicle composed of a silicone surfactant and the stabilization thereof.

### [BACKGROUND OF THE INVENTION]

Among amphiphilic compounds with both hydrophilicity and hydrophobicity, there are compounds, such as phospholipids, that form a bilayer membrane (lamellar phase) spherical structure in an aqueous phase. Such bilayer membrane structures are called liposomes or vesicles. An aqueous component can be stably incorporated inside the vesicle, and an oil component can be stably incorporated in the vesicle membrane. For example, if oily perfume is incorporated in the vesicle membrane, the perfume can be solubilized in an aqueous formulation, and the rate of perfume volatilization can be restricted. As a result, the scent can be retained for a longer period. Because of this merit, an application to fields such as pharmaceuticals, cosmetics, and food is expected.

In recent years, silicone surfactants have been reported as amphiphilic compounds that can form such vesicles (for example, refer to patent literatures 1 to 3). The characteristics of the vesicles formed of a silicone surfactant are, for example, that the vesicles can be more easily prepared in comparison with the case in which other vesicle forming surfactants are used. However, the vesicles prepared with the use of a silicone surfactant cannot stably retain, over a long period, a perfume component in the vesicle bilayer membrane, and the perfume component separates into the water phase. Thus, the commercial manufacturing of a perfume-incorporated vesicle composition could not be achieved.

It is described, in non-patent literature 1, that if a perfume is added after the preparation of silicone surfactant vesicles and a high shear force is applied (post-load method), the vesicles are temporarily broken and the perfume can be retained when the vesicles are restructured. However, as described in non-patent literature 1, there were quality issues in that the homogeneity is lost and a complicated structure forms. In addition, there was a production process problem in that a high energy is necessary for the application of a high shear force. It is also explicitly stated, in non-patent literature 1, that the solubilization cannot be achieved by the pre-load method, in which a perfume is added before the vesicle formation.

[Patent Literature 1] Japanese Unexamined Patent Publication H07-323222
[Patent Literature 2] Japanese Unexamined Patent Publication H08-239475
[Patent Literature 3] Japanese Unexamined Patent Publication H09-175930
[Non-Paten Literature 1] Lin S. B., Postiaux S., Thompson J., Novel Silicone Vesicles for Delivery of Cosmetic Actives, Proceeding of The 24th IFSCC congress, Osaka, 19B60, 2006

### [DISCLOSURE OF THE INVENTION]

### [PROBLEM TO BE SOLVED BY THE INVENTION]

The present invention was made in view of the above-described problems of the conventional art, and the objects are to provide a vesicle-containing composition wherein a perfume component is stably incorporated in the vesicle bilayer membrane composed of a silicone surfactant and to provide a simple production method of the vesicle-containing composition wherein a perfume component is stably incorporated.

### [MEANS TO SOLVE THE PROBLEM]

The present inventors have diligently studied to solve the above-described problems of the conventional art. As a result, the present inventors have found that a vesicle-containing composition wherein a perfume is stably incorporated in the vesicle bilayer membrane can be obtained by mixing the beforehand mixed oil component containing a silicone oil and a perfume with a silicone surfactant solution of one or more selected from the group consisting of ethanol, propylene glycol, dipropylene glycol, and 1,3-butylene glycol, and by further mixing with an aqueous formulation containing water and forming the vesicles of the silicone surfactant, thus leading to completion of the present invention.

That is, the vesicle-containing composition of the present invention is characterized in that the vesicle-containing composition comprising; (A) a perfume; (B) a silicone oil; (C) a silicone surfactant; (D) one or more selected from the group consisting of ethanol, propylene glycol, dipropylene glycol, and 1,3-butylene glycol; and (E) water; wherein the (C) silicone surfactant forms vesicles and 95 mass % or higher of the (A) perfume and the (B) silicone oil in the composition are present in the vesicle bilayer membrane.

In the above-described vesicle-containing composition, it is preferable that the mixing ratio of the (A) perfume and the (B) silicone oil is (A):(B) = 5:95 to 70:30 in the mass ratio. In addition, in the above-described vesicle-containing composition, it is preferable to further comprise (F) a water-soluble low-molecular-weight surfactant.

In addition, in the above-described vesicle-containing composition, it is preferable that the (B) silicone oil is octamethylcyclotetrasiloxane and/or decamethylcyclopentasiloxane.

In addition, in the above-described vesicle-containing composition, it is preferable that the (C) silicone surfactant is polyoxyalkylene-modified silicone represented by the below-described general formula (1), 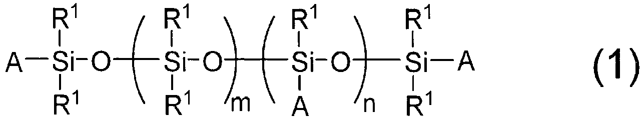 in the formula (1); R¹ is a hydrogen atom or an alkyl group having 1 to 6 carbon atoms, as for A, at least one of As is a polyoxyalkylene group represented by the formula: -(CH₂)ₐ-(C₂H₄O)_{b}-(C₃H₆O)_{c}-R², and other As are hydrogen atom(s) or alkyl group(s) having 1 to 6 carbon atoms, the number m is an integer of 1 to 200, and n is an integer of 0 to 50; and in the formula: -(CH₂)ₐ-(C₂H₄O)_{b}-(C₃H₆O)_{c}-R²; R² is a hydrogen atom or an alkyl group having 1 to 6 carbon atoms, a is an integer of 1 to 6, b is an integer of 0 to 50, c is an integer of 0 to 50, and b + c is at least 5 or higher. In addition, in the above-described vesicle-containing composition, it is preferable that the (F) water-soluble low-molecular-weight surfactant is a surfactant having a molecular weight of less than 2000. In addition, in the above-described vesicle-containing composition, it is preferable that the (D) component is one or more selected from the group consisting of propylene glycol, dipropylene glycol, and 1,3-butylene glycol.

In addition, the production method of the vesicle-containing composition of the present invention is characterized in that the production method comprising; an oil component mixing step, wherein oil components containing (A) a perfume and (B) a silicone oil are mixed, and a vesicle formation step, wherein the oil component mixture obtained in the above-described step is mixed with (C) a silicone surfactant and (D) one or more selected from the group consisting of ethanol, propylene glycol, dipropylene glycol, and 1,3-butylene glycol, and further mixed with the aqueous formulation containing (E) water to form vesicles of the (C) silicone surfactant, wherein 95 mass % or higher of the (A) perfume and the (B) silicone oil in the composition are present in the vesicle bilayer membrane.

In addition, in the production method of the above-described vesicle-containing composition, it is preferable that the mixing ratio of the (A) perfume and the (B) silicone oil is (A):(B) = 5:95 to 70:30 in the mass ratio. In addition, in the production method of the above-described vesicle-containing composition, it is preferable that (F) a water-soluble low-molecular-weight surfactant is further added and mixed after the vesicle formation step.

In addition, the cosmetic of the present invention is characterized by consisting of the above-described vesicle-containing composition.

### [EFFECT OF THE INVENTION]

According to the present invention, a vesicle-containing composition, wherein perfume is stably incorporated in the vesicle bilayer membrane, can be obtained by beforehand mixing the oil components containing a silicone oil and perfume, subsequently mixing this mixture with (C) a silicone surfactant and (D) one or more selected from the group consisting of ethanol, propylene glycol, dipropylene glycol, and 1,3-butylene glycol, and further mixing with an aqueous formulation containing (E) water, thus forming vesicles of the (C) silicone surfactant.

### [BEST MODE FOR CARRYING OUT THE INVENTION]

Hereinafter, a preferable embodiment of the present invention is described. The vesicle-containing composition of the present invention is characterized in that the vesicle-containing composition comprising; (A) a perfume; (B) a silicone oil; (C) a silicone surfactant; (D) one or more selected from the group consisting of ethanol, propylene glycol, dipropylene glycol, and 1,3-butylene glycol; and (E) water; wherein the (C) silicone surfactant forms vesicles and 95 mass % or higher of the (A) perfume and the (B) silicone oil in the composition are present in the vesicle bilayer membrane.

### (A) Perfumes

The (A) perfume used in the present invention is not limited in particular so far as it is a perfume component that is normally used in the fields of cosmetics and pharmaceuticals. Here, the (A) perfume is a mixture of various perfume components such as natural perfumes that are highly volatile oil components isolated from plants, synthetic perfumes such as terpenes, and essential oils, which are blended in accordance with the intended objective. As perfume components, there are natural perfumes and synthetic perfumes. Examples of natural perfumes include plant perfumes isolated from flowers, leaves, wood, fruit rind, etc.; and animal perfumes such as musk and civet. Examples of synthetic perfumes include hydrocarbons such as monoterpenes, alcohols such as aliphatic alcohols and aromatic alcohols, aldehydes such as terpene aldehydes and aromatic aldehydes, ketones such as alicyclic ketones, esters such as terpene esters, lactones, phenols, oxides, nitrogen-containing compounds, and acetals. These perfume components may be used alone; however, they are normally used after being suitably blended in accordance with the intended objective.

The amount of the (A) perfume is not limited in particular. However, the amount is preferably 0.01 to 5 mass % of the total amount of the composition, and more preferably 0.05 to 2 mass %. If the amount of the (A) perfume is small, a satisfactory scent may not be obtained. On the other hand, if the amount is too large, the scent may become too strong, or a negative effect to the feeling in use, such as stickiness, may be generated when the vesicle-containing composition is used as an external preparation.

### (B) Silicone oils

The (B) silicone oil used in the present invention is not limited in particular so far as the silicone oil is an oil component having a polysiloxane structure. The structure can be linear or cyclic and the property can be volatile or nonvolatile. Specific examples of the (B) silicone oil include linear silicones such as dimethylpolysiloxane, methylphenylpolysiloxane, and methylhydrogenpolysiloxane; and cyclic silicones such as octamethylcyclotetrasiloxane, decamethylcyclopentasiloxane, and dodecamethylcyclohexasiloxane. These (B) silicone oils may be used either alone or in combination of two or more.

Among these (B) silicone oils, volatile cyclic silicone oils, specifically octamethylcyclotetrasiloxane and decamethylcyclopentasiloxane can be preferably used. With the use of these volatile cyclic silicone oils, the more perfume component can be incorporated in the vesicle bilayer membrane. In addition, when the vesicle-containing composition is used as an external preparation, there is less stickiness and an excellent feeling in use can be achieved.

The amount of the (B) silicone oil is not limited in particular. However, the amount is preferably 0.1 to 1.0 mass % of the total amount of the composition, and more preferably 0.2 to 0.4 mass %. If the amount of the (B) silicone oil is small, the perfume component cannot exist in the vesicle bilayer membrane, and a phase separation may occur from the water phase. On the other hand, if the amount is too large, the vesicle stability may be poor.

In the vesicle-containing composition of the present invention, the mixing ratio of the (A) perfume and the (B) silicone oil is preferably (A):(B) = 5:95 to 70:30 in the mass ratio, and more preferably (A):(B) = 10:90 to 30:70. If the mixing ratio of the (A) perfume and the (B) silicone oil is within the above-described range, the (A) perfume and the (B) silicone oil can be effectively incorporated in the vesicle bilayer membrane.

### (C) Silicone surfactants

The (C) silicone surfactant used in the present invention is not limited in particular so far as the surfactant has a polysiloxane structure for the hydrophobic group. Specific examples of the (C) silicone surfactant include polyoxyalkylene-modified silicones represented by the below-described general formula (1).

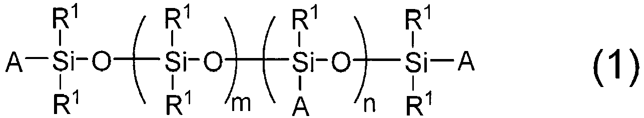

In the formula (1), R¹ is a hydrogen atom or an alkyl group having 1 to 6 carbon atoms. As for A, at least one of them is a polyoxyalkylene group represented by the formula: -(CH₂)ₐ-C₂H₄O)_{b}-(C₃H₆O)_{c}-R² (in the formula, R² is a hydrogen atom or an alkyl group having 1 to 6 carbon atoms, a is an integer of 1 to 6, b is an integer of 0 to 50, c is an integer of 0 to 50, and b + c is at least 5 or higher), and other As are hydrogen atom(s) or alkyl group(s) having 1 to 6 carbon atoms. The number m is an integer of 1 to 200, and n is an integer of 0 to 50.

In the above-described general formula (1), R¹ is a side chain on the backbone polysiloxane structure, and it is a hydrogen atom or an alkyl group having 1 to 6 carbon atoms. These may be either identical to or different from each other. For example, when all R¹s are methyl groups, the structure is a dimethylpolysiloxane structure, and when R¹s are a methyl group and a phenyl group, the structure is a methylphenylpolysiloxane structure. A is a location where a polyoxyalkylene group is introduced on the backbone of the polysiloxane structure, and at least one of them is a polyoxyalkylene group represented by the formula: -(CH₂)ₐ-(C₂H₄O)_{b}-(C₃H₆O)_{c}-R² (in the formula, R² is a hydrogen atom or an alkyl group having 1 to 6 carbon atoms, a is an integer of 1 to 6, b is an integer of 0 to 50, c is an integer of 0 to 50, and b + c is at least 5 or higher).

In the above-described general formula (1), when a portion of As is the above-described polyoxyalkylene group, other As can be a hydrogen atom or an alkyl group having 1 to 6 carbon atoms. For example, when two terminal As are polyoxyalkylene groups, it is an ABA-type polyoxyalkylene-modified silicone. On the other hand, when only the non-terminal As are polyoxyalkylene groups, it is a pendant-type polyoxyalkylene-modified silicone. The polyoxyalkylene group can be any one of the following: polyoxyethylene group, polyoxypropylene group, and polyoxyethylene/polyoxypropylene group. The number of moles of the non-substituted polysiloxane structure m is 1 to 200. The number of moles of the polyoxyalkylene-substituted polysiloxane structure n is 0 to 50. When n is 0, it is necessary that either one or both of the two terminal As are polyoxyalkylene groups.

Specific examples of the (C) silicone surfactant used in the present invention include polyoxyethylene (12 mol) modified dimethylpolysiloxane (pendant-type polyoxyalkylene-modified silicone wherein the side chain methyl group of a linear dimethylpolysiloxane is replaced with a polyoxyethylene (12 mol) group), polyoxyethylene (8 mol) modified dimethylpolysiloxane, and polyoxyethylene (20 mol) modified dimethylpolysiloxane. Other examples include an ABA-type polyoxyethylene-methylsiloxane-polyoxyethylene block copolymer. In these polyoxyethylene-modified silicones, it is preferable that the molecular weight of ethylene oxide in the total molecular weight is 20 to 60%. The (C) silicone surfactant used in the present invention may be produced by a publicly known method, or commercial products may be used. Examples of commercial silicone surfactants include SH3772M, SH3773M, SH3775M (all manufactured by Dow Corning Silicone Co., Ltd.), and IM-22 (manufactured by Wacker Chemical Corp.). These (C) silicone surfactants may be used either alone or in combination of two or more.

The (C) silicone surfactant is contained as vesicles in the vesicle-containing composition of the present invention. The formation of vesicles can be easily carried out by a publicly known method. For example, the vesicles of the (C) silicone surfactant in an aqueous formulation can be formed by mixing and stirring the (C) silicone surfactant and (D) one or more selected from the group consisting of ethanol, propylene glycol, dipropylene glycol, and 1,3-butylene glycol in the aqueous formulation. The vesicle particle size is not limited in particular. However, the vesicle particle size is normally about 20 to 500 nm and preferably 50 to 200 nm.

The HLB of the (C) silicone surfactant is not limited in particular. However, from the viewpoint of vesicle formation, the HLB is preferably 4 to 12 and more preferably 6 to 9. If the HLB of the (C) silicone surfactant deviates from the above-described range, it will become difficult to form stable vesicles; in addition, there are cases in that the (A) perfume and the (B) silicone oil cannot be effectively incorporated into the vesicle bilayer membrane.

The amount of the (C) silicone surfactant is not limited in particular so far as the quantity is enough for the formation of vesicles. However, the quantity is preferably 0.1 to 10 mass % of the total amount of the composition, and more preferably 0.2 to 5.0 mass %. If the amount of the (C) silicone surfactant is small, the effect of vesicle formation may not be achieved. On the other hand, if the amount is too large, the vesicle stability may be poor. In the vesicle-containing composition of the present invention, it is preferable that the mixing ratio of the (B) silicone oil and the (C) silicone surfactant is (B):(C) = 1:0.1 to 1:0.4 in the mass ratio.

### (D) Ethanol and others

The (D) component is one or more selected from the group consisting of ethanol, propylene glycol, dipropylene glycol, and 1,3-butylene glycol. There is no problem in the use of ethanol because the perfume component can be stably incorporated into the vesicles; however, there is concern about the irritation to the skin when a large amount of ethanol is blended. In addition, the scent emanation is disturbed because of a unique irritating smell; thus the amount of ethanol should better be small. As the (D) component, propylene glycol, dipropylene glycol, or 1,3-butylene glycol is preferable, and dipropylene glycol is especially preferable. The amount of the (D) component is not limited in particular; however, the amount is preferably 0.1 to 15 mass % of the total amount of the composition, and more preferably 1 to 5 mass %. If the amount of the (D) component deviates from the above-described range, the vesicles of the (C) silicone surfactant may not be formed.

### (E) Water

The amount of (E) water is not limited in particular; however, the amount is preferably 70 to 90 mass % of the total amount of the composition, and more preferably 80 to 95 mass %. If the amount of (E) water is small, vesicles may not be formed. On the other hand, if the amount of water is too large, the relative amounts of the (A) perfume and the (B) silicone oil decrease in the composition. As a result, the blending effect of the (A) and (B) components may not be achieved.

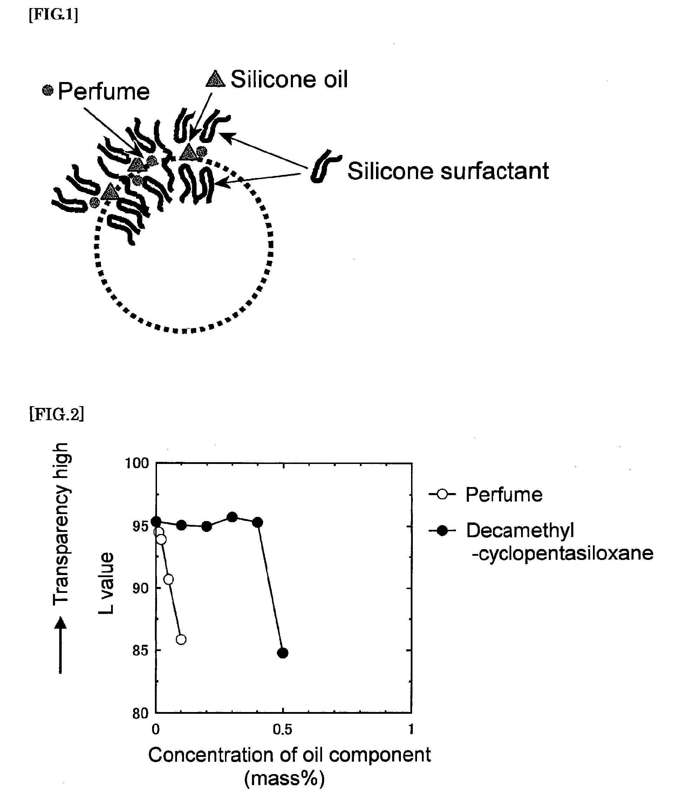

The vesicle-containing composition of the present invention comprises the above-described (A) to (E) components, and the (A) perfume and the (B) silicone oil are present in the vesicle bilayer membrane that is formed with the (C) silicone surfactant. In the vesicles, terminal hydrophobic groups of the surfactant molecules normally come close to each other. Thus, spherical vesicles are formed, with a continuous structure of a bilayer membrane, by keeping the hydrophobic groups on the inside and the hydrophilic groups on the outside. As shown in Fig. 1, in the vesicle-containing composition of the present invention, the (A) perfume and the (B) silicone oil are present inside the bilayer membrane formed with the (C) silicone surfactant, namely in the space inside the membrane wherein the hydrophobic groups of the (C) silicone surfactant are facing to each other. The entire amount of the (A) perfume and the (B) silicone oil need not exist in the vesicle bilayer membrane. However, 95 mass % or higher of the (A) perfume and the (B) silicone oil that are contained in the composition is present in the vesicle bilayer membrane.

### (F) Water-soluble low-molecular-weight surfactants

In the vesicle-containing composition of the present invention, the stability of the vesicles formed with the (C) silicone surfactant is improved by also adding (F) a water-soluble low-molecular-weight surfactant.

The (F) water-soluble low-molecular-weight surfactant used in the present invention is not limited in particular so far as the surfactant is other than the (C) silicone surfactant, water-soluble, and of low molecular weight. Here, the low molecular weight specifically means molecular weights less than 3000. If a surfactant with a molecular weight of 3000 or higher is used, a satisfactory vesicle stabilizing effect cannot be achieved. It is especially preferable that the molecular weight of the surfactant is less than 2000. Specific examples of the (F) water-soluble low-molecular-weight surfactant include anionic surfactants such as sodium polyoxyethylene (2 mol) lauryl ether carboxylate, sodium polyoxyethylene (2 mol) lauryl ether sulfonate, and polyoxyethylene (2 mol) lauryl ether sulfuric acid; branched nonionic surfactants such as polyoxyethylene (24 mol)-polyoxypropylene (13 mol) 2-decyltetradecyl ether, polyoxyethylene (60 mol) hydrogenated castor oil, and polyoxyethylene (30 mol) octyldodecyl ether; and linear nonionic surfactant such as polyoxyethylene (20 mol) cetyl ether. Among these, in the case of anionic surfactants, a surfactant of molecular weight less than 500 can be especially preferably used. In the case of nonionic surfactants, a surfactant of molecular weight less than 2000 can be especially preferably used. The water-soluble low-molecular-weight surfactants can be used alone or more than one can be arbitrarily selected and used in combination.

The amount of the (F) water-soluble low-molecular-weight surfactant is preferably 0.05 to 2.0 mass % of the total amount of the composition, and more preferably 0.1 to 0.3 mass %. If the amount of the (E) water-soluble low-molecular-weight surfactant is small, a satisfactory vesicle stabilizing effect may not be achieved. On the other hand, if the amount is too large, the vesicles start to be solubilized, and rather a negative effect may be produced.

In addition, when the (F) water-soluble low-molecular-weight surfactant is an anionic surfactant, it is preferable that the mixing ratio of the (C) silicone surfactant and the (F) anionic surfactant is 1:0.05 to 1:0.3. When the (F) water-soluble low-molecular-weight surfactant is a branched nonionic surfactant, it is preferable that the mixing ratio of the (C) silicone surfactant and the (F) branched nonionic surfactant is 1:0.05 to 1:1. When the (F) water-soluble low-molecular-weight surfactant is a linear nonionic surfactant, it is preferable that the mixing ratio of the (C) silicone surfactant and the (F) linear nonionic surfactant is 1:0.05 to 1:0.5. If the mixing ratio of the (C) silicone surfactant and the (F) water-soluble low-molecular-weight surfactant deviates from the above-described range, the vesicle stabilizing effect may not be achieved.

### Production method

The production method of the vesicle-containing composition of the present invention comprises an oil component mixing step, wherein the oil component containing the (A) perfume and the (B) silicone oil is mixed, and a vesicle formation step, wherein the oil component mixture obtained in the above-described step is mixed with the (C) silicone surfactant and (D) one or more selected from the group consisting of ethanol, propylene glycol, dipropylene glycol, and 1,3-butylene glycol, and further mixed with the aqueous formulation containing (E) water to form vesicles of the (C) silicone surfactant.

In the production method of the vesicle-containing composition of the present invention, oil components containing the (A) perfume and the (B) silicone oil are mixed beforehand. By mixing the (A) perfume and the (B) silicone oil beforehand, the (A) perfume is easily incorporated into the vesicle bilayer membrane. In this oil component mixing step, other oil components normally used in cosmetics, pharmaceuticals, etc. can be added, in addition to (A) and (B) components, in the range that does not affect the vesicle formation and stability thereof.

Subsequently, an oil component mixture containing (A) and (B) components obtained in the above-described step is mixed with the (C) silicone surfactant and (D) one or more selected from the group consisting of ethanol, propylene glycol, dipropylene glycol, and 1,3-butylene glycol, and further mixed with the aqueous formulation containing (E) water. Vesicles are easily formed by mixing and stirring the (C) silicone surfactant and the (D) ethanol etc. into the aqueous formulation. Here, (C) and (D) components can be individually added to and mixed with the oil component mixture. The aqueous formulation is not limited in particular so far as water and/or aqueous solvent is the main medium of the formulation. The components normally used in cosmetics, pharmaceuticals, etc. can be added in the range that does not affect the vesicle formation and stability thereof.

Thus, the (A) perfume and the (B) silicone oil are incorporated in the vesicles of the (C) silicone surfactant and are present in the vesicle bilayer membrane by mixing a beforehand prepared oil mixture containing the (A) perfume and the (B) silicone oil with the (C) silicone surfactant and (D) one or more selected from the group consisting of ethanol, propylene glycol, dipropylene glycol, and 1,3-butylene glycol, and by mixing with the aqueous formulation containing (E) water. If the (A) perfume and the (B) silicone oil are not mixed beforehand and individually mixed with the aqueous formulation containing (C) and (D) components and (E) water, almost only the (B) silicone oil is incorporated in the vesicle bilayer membrane, and much of the (A) perfume is separated into the water phase. Even if the (A) perfume is incorporated in the vesicle bilayer membrane, the separation tends to take place with time.

In addition, in the production method of the vesicle-containing composition of the present invention, the vesicles of the (C) silicone surfactant can be stabilized by adding and mixing a (F) water-soluble low-molecular-weight surfactant. However, it is necessary to add the (F) water-soluble low-molecular-weight surfactant after the vesicle formation step. If the (F) water-soluble low-molecular-weight surfactant is added before the vesicle formation step, the (A) perfume and the (B) silicone oil are emulsified with the (F) water-soluble low-molecular-weight surfactant. As a result, the (A) and (B) components cannot be incorporated into the vesicle bilayer membrane. In addition, the (F) water-soluble low-molecular-weight surfactant affects the formation of vesicles of the (C) silicone surfactant, and a negative effect may impact vesicle stability.

The vesicle-containing composition of the present invention can suitably used, for example, as cosmetics. When the composition is used as cosmetics, other components normally used in pharmaceuticals and cosmetics can be added in addition to the above-described essential components so far as the amount is in the range that does not affect the vesicle formation and stability thereof. These other formulation components may be suitably added into the oil component mixture or into the aqueous formulation either before or after vesicle formation. In addition, for example, a microcapsule composition wherein a chemical component is present only in the inner phase can be prepared by forming vesicles by blending the chemical component into an aqueous formulation and then replacing the outer phase.

The use of the cosmetics of the present invention is not limited in particular, and they are suitably used, for example, as a lotion and as a hair liquid.

### [EXAMPLE 1]

Hereinafter, the present invention will be described in further detail with reference to the examples. However, the present invention is not limited by these examples. Hereinafter, the amount is expressed in mass % unless otherwise noted.

In order to investigate the uptake quantity of an oil component into the vesicles of silicone surfactant, the present inventors determined the uptake quantity of the perfume (limonene:linalol:benzyl acetate = 1:1:1) and decamethylcyclopentasiloxane into the formed vesicles of polyoxyethylene (12 mol) dimethylpolysiloxane (HLB = 8) as the silicone surfactant. The test method was as follows. The results are shown in Table 1 and Fig. 2.

### <Test method>

The silicone surfactant (polyoxyethylene (12 mol) dimethylpolysiloxane (HLB = 8)) and the oil component (perfume (limonene:linalol:benzyl acetate =1:1:1) or decamethylcyclopentasiloxane) were added to and mixed with ethanol. Subsequently, vesicles were formed by diluting with water until the concentration of the silicone surfactant became 1.0 mass %. Various vesicle-containing compositions were obtained by varying the amount of the added oil component so that the respective final concentrations of the oil component would be 0, 0.1, 0.2, 0.3, 0.4, and 0.5 mass %. The transparency (L value) was measured with a Color-Eye 7000A spectrophotometer (manufactured by GretagMacbeth) for the obtained vesicle-containing compositions.

**[Table 1]**

| Concentration of Oil Component (mass %) | Transparency (L value) | |
|---|---|---|
| | Perfume | Decamethylcyclopentasiloxane |
| 0 | 95.32 | 95.32 |
| 0.01 | 94.49 | - |
| 0.02 | 93.89 | - |
| 0.05 | 90.68 | - |
| 0.1 | 85.85 | 95.04 |
| 0.2 | - | 94.95 |
| 0.3 | - | 95.70 |
| 0.4 | - | 95.29 |
| 0.5 | - | 84.78 |

As shown in Table 1 and Fig. 1, when the perfume was used as the oil component, the transparency drastically decreased by adding only a small amount of the perfume. By the observation with an optical microscope, it was confirmed that the perfume was segregated as oil droplets with the size of about 1µm. Thus, it is clear that the perfume was not at all incorporated into the vesicles. On the other hand, when decamethylcyclopentasiloxane was used as the oil component, a decrease in the transparency, for the 1.0 mass % silicone surfactant, did not take place up to about 0.4 mass % of the oil component. In addition, no oil droplets were observed by optical microscope observation. Thus, it is possible to incorporate the oil component into the vesicles up to this concentration.

In addition, the present inventors investigated, in the same way as the above-described test, the maximum uptake quantity of various oil components into the silicone surfactant vesicles. The maximum uptake quantity of an oil component is the inflection point at which the transparency decreases when the oil component is increased gradually. The results are shown in Table 2.

**[Table 2]**

| Oil Component | Maximum uptake quantity (mass %) |
|---|---|
| Liquid paraffin | 0 |
| Cetyl ethylhexanoate | 0 |
| Glyceryl tri-2-ethylhexanoate | 0 |
| Dimethylsiloxane (6 cps) | 0.1 |
| Methylphenylsiloxane | 0.3 |
| Decamethylcyclopentasiloxane | 0.4 |

As shown in Table 2, when various hydrocarbon oils such as liquid paraffin and ester oil were used, these oils could not be incorporated into the vesicles. On the other hand, when various silicone oils such as dimethylsiloxane were used, for the 1.0 mass % silicone surfactant, it was possible to incorporate about 0.1 to 0.4 mass % of the oil component into the vesicles.

In view of the above-described test results, the present inventors investigated the uptake of the oil component mixture, which was beforehand obtained by mixing perfume and a silicone oil, into the vesicles of a silicone surfactant. As the perfume, a mixture of limonene:linalol:benzyl acetate = 1:1:1 was used in the same way as the above-described test. Various compositions tested and the results of transparency evaluation are shown in Table 3. The measurement conditions of transparency were the same as the above-described test.

**[Table 3]**

| | Test Example 1 | Test Example 2 | Test Example 3 |
|---|---|---|---|
| POE(12)dimethylpolysiloxane (HLB=8) | 1.0 | 1.0 | 1.0 |
| Ethanol | 1.0 | 1.0 | 1.0 |
| Decamethylcyclopentasiloxane | - | 0.2 | - |
| Methylphenylsiloxane | - | - | 0.1 |
| Perfume | - | 0.02 | 0.02 |
| Ion-exchanged water | Balance | Balance | Balance |
| Appearance | Transparent | Transparent | Transparent |
| Transparency (L value) | 95.3 | 95.4 | 95.2 |

(Production method) The perfume and respective silicone oils were mixed, and this mixture was mixed with the silicone surfactant dissolved in ethanol. Then, to this was added ion-exchanged water (until the silicone surfactant became 1.0 mass %) and mixed to form vesicles.

As shown in Table 3, no difference in transparency was observed between the vesicle-containing composition of Test Example 2, wherein a mixture of the perfume and decamethylcyclopentasiloxane was used, and the vesicle-containing composition of Test Example 1, wherein no oil was blended. Therefore, almost the entire amount of the oil component mixture containing the perfume is incorporated into the vesicles of the silicone surfactant. In Test Example 3, wherein methylphenylsiloxane was used, the oil mixture containing the perfume is also incorporated in the vesicles, as is the case in Test Example 2. From the above-described results, it was found that the perfume that cannot be incorporated alone into the vesicles can be incorporated into the vesicles of silicone surfactant with the use of the oil component mixture wherein the perfume and silicone oil are mixed.

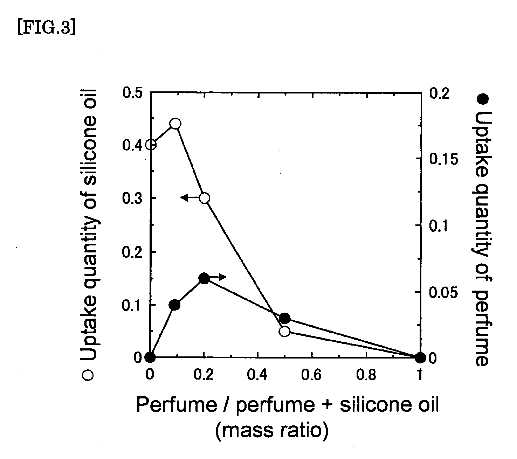

In order to investigate the preferable mixing ratio of the perfume and silicone oil, the present inventors prepared vesicle-containing compositions, in the same way as the above-described test, with the use of mixtures with various mixing ratios of perfume/silicone oil, and investigated the respective uptake quantities of the perfume and silicone oil. In the same way as the above-described test, a mixture of limonene:linalol:benzyl acetate = 1:1:1 was used as the perfume, decamethylcyclopentasiloxane was used as the silicone oil, and polyoxyethylene (12 mol) dimethylpolysiloxane (HLB = 8) was used as the silicone surfactant. The concentration of the silicone surfactant was adjusted to the final concentration of 1.0 mass %. The maximum uptake quantity of oil components was determined by measuring the transparency (L value) with a Color-Eye 7000A spectrophotometer (manufactured by GretagMacbeth). The results are shown in Table 4 and Fig. 3.

**[Table 4]**

| Perfume/(Perfume + Silicone oil) (mass ratio) | Uptake quantity of silicone oil | Uptake quantity of perfume |
|---|---|---|
| 0 | 0.40 | 0 |
| 0.09 | 0.44 | 0.04 |
| 0.20 | 0.30 | 0.06 |
| 0.50 | 0.05 | 0.03 |
| 1.00 | 0 | 0 |

As shown in Table 4 and Fig. 3, when the perfume was gradually added into the silicone oil, the uptake quantity of the perfume into the vesicles increased with the added amount of the perfume. At an approximate mixing ratio of perfume : silicone oil = 20:80, the uptake quantity of the perfume became the maximum. After further adding the perfume, when the mixing ratio of perfume and the silicone was half and half, the uptake quantity of the perfume decreased to about a half of the maximum value. When the entirety became the perfume, the uptake quantity of the perfume became zero.
In order to effectively incorporate the perfume into the vesicles, it is considered, from the results of Fig. 3, that the mixing ratio of the perfume and the silicone oil is preferably about 5:95 to 70:30 in the mass ratio, and more preferably about 10:90 to 30:70.

In order to stabilize the vesicle-containing composition wherein the perfume is incorporated as described above, the present inventors tried the preparation of a vesicle-containing composition wherein a water-soluble low-molecular-weight surfactant was added. Here, sodium polyoxyethylene (2 mol) lauryl ether carboxylate was used as the water-soluble low-molecular-weight surfactant, and a mixture of limonene:linalol:benzyl acetate = 1:1:1 was used as the perfume in the same way as the above-described test. The measurement of the transparency was carried out twice, in the same way as the above-described test, immediately after the production and after 4 weeks at 50°C. The various compositions tested and the evaluation results of transparency are both shown in Table 5.

**[Table 5]**

| | Test Example 2 | Test Example 4 | Test Example 5 | Test Example 6 |
|---|---|---|---|---|
| POE(12)dimethylpolysiloxane (HLB=8) | 1.0 | 1.0 | 1.0 | 1.0 |
| Ethanol | 1.0 | 1.0 | 1.0 | 1.0 |
| Decamethylcyclopentasiloxane | 0.2 | 0.2 | 0.2 | 0.2 |
| Perfume | 0.02 | 0.02 | 0.03 | 0.04 |
| Sodium POE(2) lauryl ether carboxylate | - | 0.01 | 0.01 | 0.01 |
| Ion-exchanged water | Balance | Balance | Balance | Balance |
| Appearance | Transparent | Transparent | Transparent | Transparent |
| Transparency (L value) | | | | |
| Immediately after the production | 95.42 | 97.07 | 97.21 | 96.31 |
| After 4 weeks at 50 °C | 62.05 | 97.13 | 97.19 | 96.17 |

(Production method) The perfume and the silicone oils were mixed, and this mixture was mixed with the silicone surfactant dissolved in ethanol. Then, ion-exchanged water was added to this and mixed to form vesicles. In addition, the water-soluble low-molecular-weight surfactant was added and mixed to the obtained vesicle-containing composition.

As shown in Table 5, in the vesicle-containing composition of Test Example 2, wherein the water-soluble low-molecular-weight surfactant is not blended, the transparency slightly decreased after 4 weeks at 50°C. It is suggested that the vesicle structure is partially collapsed at the high temperature with time. On the other hand, in the vesicle-containing composition of Test Examples 4 to 6, wherein 0.01 mass % of water-soluble low-molecular-weight surfactant was added and blended, the transparency did not decrease after 4 weeks at 50 °C. Thus, it is clear that the vesicle structure was stably maintained at the high temperature with time.

In addition, the present inventors investigated the property change due to differences of the (D) component. Here, sodium polyoxyethylene (2 mol) lauryl ether carboxylate was used as the water-soluble low-molecular-weight surfactant, and a mixture of limonene:linalol:benzyl acetate = 1:1:1 was used as the perfume in the same way as the above-described test. The measurement of the transparency was carried out twice, in the same way as the above-described test, immediately after the production and after 4 weeks at 50°C. The test of scent emanation was performed by professional panelists. The evaluation criteria were as follows. The various compositions tested and the evaluation results, for the scent emanation test and transparency, are both shown in Table 6.

### <Evaluation method of scent emanation>

The scent emanation when applied on the face was evaluated by five professional panelists.
⊚: 5 panelists evaluated that the scent emanation was good.
○: 4 panelists evaluated that the scent emanation was good.
Δ: 3 panelists evaluated that the scent emanation was good.
X: 2 or less panelists evaluated that the scent emanation was good.

**[Table 6]**

| | Test Example 7 | Test Example 8 | Test Example 9 |
|---|---|---|---|
| POE(12)dimethylpolysiloxane (HLB=8) | 1.0 | 1.0 | 1.0 |
| Ethanol | 4.0 | 2.0 | - |
| Dipropylene glycol | - | 2.0 | 4.0 |
| Decamethylcyclopentasiloxane | 0.2 | 0.2 | 0.2 |
| Perfume | 0.02 | 0.02 | 0.03 |
| Sodium POE(2) lauryl ether carboxylate | 0.01 | 0.01 | 0.01 |
| Ion-exchanged water | Balance | Balance | Balance |
| Appearance | Transparent | Transparent | Transparent |
| Scent emanation | ○ | ⊚ | ⊚ |
| Transparency (L value) | | | |
| Immediately after the production | 97.4 | 97.7 | 97.0 |
| After 4 weeks at 50°C | 97.5 | 97.3 | 96.9 |

As shown in Table 6, in Test Example 7, wherein 4 mass % of ethanol was blended, the transparency was excellent; however, the scent emanation tended to be somewhat impaired. On the other hand, in both Test Example 8, wherein 2 mass % of dipropylene glycol and 2 mass % of ethanol were blended, and Test Example 9, wherein 4 mass % of dipropylene glycol was blended, the scent emanation was not impaired, and a vesicle-containing composition having excellent transparency was obtained.

Hereinafter, cosmetic formulation examples of the vesicle-containing composition of the present invention will be listed; however, the present invention is not limited by these examples. All the obtained compositions were excellent in vesicle formation capability and vesicle stability.

| Formulation Example 1 | (mass %) |
|---|---|
| (1) POE(12)dimethylpolysiloxane (HLB=6) | 0.5 |
| (2) Ethanol | 7 |
| (3) Glycerin | 5 |
| (4) Dipropylene glycol | 2 |
| (5) Citric acid | 0.1 |
| (6) Sodium citrate | 0.4 |
| (7) Ascorbic acid-2-glucoside | 2 |
| (8) Potassium hydroxide | 1 |
| (9) Phenylsiloxane | 0.1 |
| (10) Benzyl acetate | 0.02 |
| (11) Purified water | Balance |

(Production method) POE (12) dimethylpolysiloxane (HLB = 6) was dissolved in a mixture of ethanol/dipropylene glycol. Into this, a mixture of phenylsiloxane and benzyl acetate was dissolved. A lotion was obtained by adding and mixing the above solution into a water phase composed of other components.

| Formulation Example 2 | (mass %) |
|---|---|
| (1) POE(12)dimethylpolysiloxane (HLB=9) | 5 |
| (2) Ethanol | 7 |
| (3) 1,3-Butylene glycol | 10 |
| (4) Limonene | 0.1 |
| (5) Linalol | 0.1 |
| (6) Decamethylcyclopentasiloxane | 0.8 |
| (7) EDTA3Na·2H₂O | 0.1 |
| (8) Phenoxyethanol | 0.5 |
| (9) Purified water | Balance |

(Production method) POE (12) dimethylpolysiloxane (HLB = 6) was dissolved in a mixture of ethanol/1,3-butylene glycol. Into this, a mixture of decamethylcyclopentasiloxane, limonene, and linalol was dissolved. A lotion was obtained by adding and mixing the above solution into a water phase composed of other components.

| Formulation Example 3 | (mass %) |
|---|---|
| (1) POE(12)dimethylpolysiloxane (HLB=9) | 5 |
| (2) Dipropylene glycol | 7 |
| (3) Ethyl vitamin C | 1 |
| (4) Limonene | 0.1 |
| (5) Linalol | 0.1 |
| (6) Decamethylcyclopentasiloxane | 0.8 |
| (7) EDTA3Na·2H₂O | 0.1 |
| (8) Phenoxyethanol | 0.5 |
| (9) Potassium POE(2 mol) lauryl ether carboxylate | 0.5 |
| (10) Purified water | Balance |

(Production method) POE (12) dimethylpolysiloxane (HLB = 6) was dissolved in dipropylene glycol. To this, a mixture of decamethylcyclopentasiloxane, limonene, and linalol was dissolved. The above solution was added and mixed into a water phase composed of other components except for potassium POE (2 mol) lauryl ether carboxylate. Then, a lotion was obtained by adding potassium POE (2 mol) lauryl ether carboxylate and stirring.

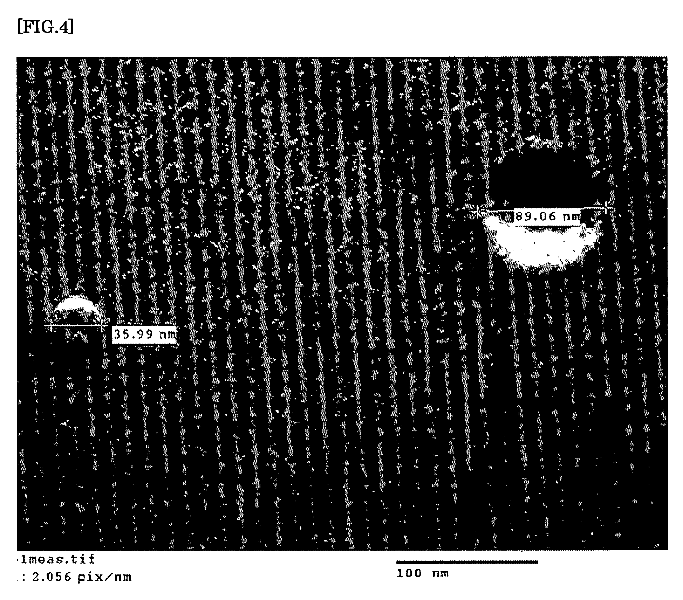

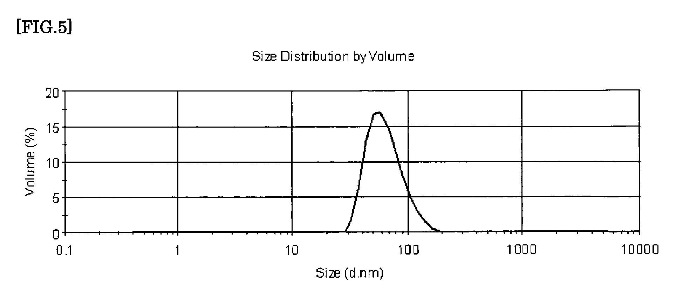

An electron micrograph of the vesicle particle of the lotion that was obtained in the above-described Formulation Example 3 is shown in Fig. 4, and the measurement results for the vesicle particle size are shown in Fig. 5. As seen in Fig. 4, the formation of vesicle particles was observed in the lotion of Formulation Example 3. In addition, as seen in Fig. 5, the average particle size of vesicle particles in the lotion of Formulation Example 3 was about 65 nm.

### [BRIEF DESCRIPTION OF THE DRAWINGS]

**[****Fig. 1****]** is an illustration of the vesicle-containing composition of the present invention.
**[****Fig. 2****]** is a summary of the transparency (L value) measurement results for the vesicle compositions that are formed with various concentrations of perfume or decamethylcyclopentasiloxane with a silicone surfactant.
**[****Fig. 3****]** is a summary of the measurement results for the uptake quantity of perfume or silicone oil into the silicone surfactant vesicle when various mixing ratios of perfume/silicone oil were used.
**[****Fig. 4****]** is an electron micrograph of vesicle particles in the lotion of Formulation Example 3.
**[****Fig. 5****]** shows the results of vesicle particle size measurement in the lotion of Formulation Example 3.

## Claims

1. A vesicle-containing composition comprising;
(A) a perfume,
(B) a silicone oil,
(C) a silicone surfactant,
(D) one or more selected from the group consisting of ethanol, propylene glycol, dipropylene glycol, and 1,3-butylene glycol, and
(E) water;
wherein the (C) silicone surfactant forms vesicles, and 95 mass % or higher of the (A) perfume and the (B) silicone oil in the composition are present in the vesicle bilayer membrane.

2. The vesicle-containing composition according to claim 1, wherein the mixing ratio of the (A) perfume and the (B) silicone oil is (A):(B) = 5:95 to 70:30 in the mass ratio.

3. The vesicle-containing composition according to claim 1 or 2, further comprising (F) a water-soluble low-molecular-weight surfactant.

4. The vesicle-containing composition according to any of claim 1 to 3, further comprising (G) a polar oil having an IOB of 0.1 to 0.4.

5. The vesicle-containing composition according to any of claim 1 to 4, wherein the (B) silicone oil is octamethylcyclotetrasiloxane and/or decamethyl-cyclopentasiloxane.

6. The vesicle-containing composition according to any of claim 1 to 5, wherein the (C) silicone surfactant is polyoxyalkylene-modified silicone represented by the below-described general formula (1), 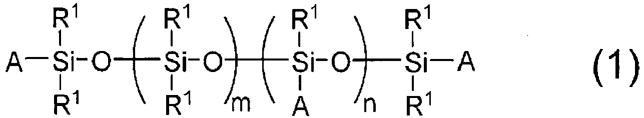 in the formula (1); R¹ is a hydrogen atom or an alkyl group having 1 to 6 carbon atoms, as for A, at least one of As is a polyoxyalkylene group represented by the formula: -(CH₂)ₐ-(C₂H₄O)_{b}-(C₃H₆O)_{c}-R², and other As are hydrogen atom(s) or alkyl group(s) having 1 to 6 carbon atoms, the number m is an integer of 1 to 200, and n is an integer of 0 to 50; and in the formula: -(CH₂)ₐ-(C₂H₄O)_{b}-(C₃H₆O)_{c}-R²; R² is a hydrogen atom or an alkyl group having 1 to 6 carbon atoms, a is an integer of 1 to 6, b is an integer of 0 to 50, c is an integer of 0 to 50, and b + c is at least 5 or higher.

7. The vesicle-containing composition according to any of claim 3 to 6, wherein the (F) water-soluble low-molecular-weight surfactant is a surfactant having a molecular weight of less than 2000.

8. The vesicle-containing composition according to any of claim 1 to 7, wherein the (D) component is one or more selected from the group consisting of propylene glycol, dipropylene glycol, and 1,3-butylene glycol.

9. The vesicle-containing composition according to any of claim 4 to 8, wherein the (G) component is one or more selected from the group consisting of glyceryl tri-2-ethylhexanoate, cetyl isooctanoate, pentaerythritol tetra-2-ethyl-hexanoate, and isononyl isononanoate.

10. A production method of a vesicle-containing composition comprising;
an oil component mixing step, wherein oil components containing (A) a perfume and (B) a silicone oil are mixed, and
a vesicle formation step, wherein the oil component mixture obtained in the above-described step is mixed with (C) a silicone surfactant and (D) one or more selected from the group consisting of ethanol, propylene glycol, dipropylene glycol, and 1,3-butylene glycol, and further mixed with the aqueous formulation containing (E) water to form vesicles of the (C) silicone surfactantₗ wherein 95 mass % or higher of the (A) perfume and the (B) silicone oil are present in the vesicle bilayer membrane in the obtained composition.

11. The production method of a vesicle-containing composition according to claim 10, wherein the mixing ratio of the (A) perfume and the (B) silicone oil is (A):(B) = 5:95 to 70:30 in the mass ratio.

12. The production method of a vesicle-containing composition according to claim 10 or 11, wherein (F) a water-soluble low-molecular-weight surfactant is further added and mixed after the vesicle formation step.

13. The production method of a vesicle-containing composition according to any of claim 10 to 12, wherein (G) a polar oil having an IOB of 0.1 to 0.4 is mixed together during the oil component mixing step.

14. The production method of a vesicle-containing composition according to any of claims 10 to 13 for producing a composition according to claims 5 to 9.

15. A cosmetic consisting of the vesicle-containing composition according to any of claim 1 to 9.

## Patentansprüche

1. Vesikelhaltige Zusammensetzung, enthaltend:
(A) einen Duftstoff,
(B) Silikonöl,
(C) ein Silikontensid,
(D) eine oder mehrere Substanzen ausgewählt aus der Gruppe bestehend aus Ethanol, Propylenglykol, Dipropylenglykol und 1,3-Butylenglykol, und
(E) Wasser
wobei das Silikontensid (C) Vesikel bildet und wobei 95 Gew.-% oder mehr des Duftstoffes (A) und des Silikonöls (B) in der Zusammensetzung in der zweischichten Membran der Vesikel vorhanden sind.

2. Vesikelhaltige Zusammensetzung gemäß Anspruch 1, wobei das Mischverhältnis des Duftstoffes (A) und des Silikonöls (B) A:B = 5:95 bis 70:30 in dem Massenverhältnis beträgt.

3. Vesikelhaltige Zusammensetzung gemäß Anspruch 1 oder 2, ferner enthaltend (F) ein wasserlösliches niedermolekulares Tensid.

4. Vesikelhaltige Zusammensetzung gemäß einem der Ansprüche 1 bis 3, ferner enthaltend (G) ein polares Öl mit einem IOB-Wert von 0,1 bis 0,4.

5. Vesikelhaltige Zusammensetzung gemäß einem der Ansprüche 1 bis 4, wobei das Silikonöl (B) Octamethylcyclotetrasiloxan und/oder Decamethylcyclopentasiloxan ist.

6. Vesikelhaltige Zusammensetzung gemäß einem der Ansprüche 1 bis 5, wobei das Silikontensid (C) polyoxyalkylenmodifiziertes Silikon ist, dargestellt durch die allgemeine Formel (1) 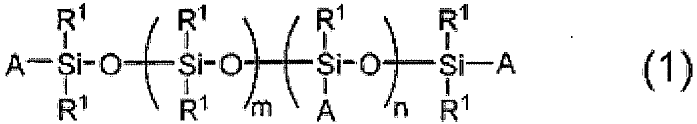 wobei in der Formel (1) R¹ bezüglich A ein Wasserstoffatom oder eine Alkylgruppe mit 1 bis 6 Kohlenstoffatomen ist, wobei mindestens ein A eine Polyoxyalkylengruppe ist, dargestellt durch die Formel -(CH₂)ₐ-(C₂H₄O)_{b}-(C₃H₆O)_{c}-R² und die anderen A ein (mehrere) Wasserstoffatom(e) oder eine (mehrere) Alkylgruppe(n) mit 1 bis 6 Kohlenstoffatomen sind, wobei m eine ganze Zahl von 1 bis 200 und n eine ganze Zahl von 0 bis 50 ist; und wobei in der Formel -(CH₂)ₐ-(C₂H₄O)_{b}-(C₃H₆O)_{c}-R² R² ein Wasserstoffatom oder eine Alkylgruppe mit 1 bis 6 Kohlenstoffatomen ist, a eine ganze Zahl von 1 bis 6 ist, b eine ganze Zahl von 0 bis 50 ist, c eine ganze Zahl von 0 bis 50 ist und b + c mindestens 5 oder größer sind.

7. Vesikelhaltige Zusammensetzung gemäß einem der Ansprüche 3 bis 6, wobei das wasserlösliche niedermolekulare Tensid (F) ein Tensid mit einem Molekulargewicht von weniger als 2000 ist.

8. Vesikelhaltige Zusammensetzung gemäß einem der Ansprüche 1 bis 7, wobei die Komponente (D) eine oder mehrere ausgewählt aus der Gruppe bestehend aus Propylenglykol, Dipropylenglykol und 1,3-Butylenglykol ist.

9. Vesikelhaltige Zusammensetzung gemäß einem der Ansprüche 4 bis 8, wobei die Komponente (G) eine oder mehrere ausgewählt aus der Gruppe bestehend aus Glyceryl-tri(2-ethylhexanoat), Cetylisooctanoat, Pentaerythritol-tetra(2-ethylhexanoat) und Isononylisononanoat ist.

10. Herstellungsverfahren für eine vesikelhaltige Zusammensetzung, umfassend:
einen Schritt des Mischens der Ölkomponenten, wobei Ölkomponenten gemischt werden, die (A) einen Duftstoff und (B) ein Silkonöl enthalten, und
einen Schritt zum Bilden der Vesikel, wobei das in dem vorstehend beschriebenen Mischschritt erhaltene Ölkomponentengemisch mit (C) einem Silikontensid und (D) einem oder mehreren Substanzen ausgewählt aus der Gruppe bestehend aus Ethanol, Propylenglykol, Dipropylenglykol und 1,3-Butylenglykol und weiter mit der wasserhaltigen Formulierung enthaltend (E) Wasser gemischt wird, um Vesikel des Silkontensids (C) zu bilden, wobei 95 Gew.-% oder mehr des Duftstoffes (A) und des Silikonöls (B) in der zweischichten Membran der Vesikel in der erhaltenen Zusammensetzung vorhanden sind.

11. Herstellungsverfahren für eine vesikelhaltige Zusammensetzung gemäß Anspruch 10, wobei das Mischverhältnis des Duftstoffes (A) und des Silikonöls (B) A:B = 5:95 bis 70:30 in dem Massenverhältnis beträgt.

12. Herstellungsverfahren für eine vesikelhaltige Zusammensetzung gemäß Anspruch 10 oder 11, wobei (F) nach dem Schritt zum Bilden der Vesikel weiter ein wasserlösliches niedermolekulares Tensid zugegeben und zugemischt wird.

13. Herstellungsverfahren für eine vesikelhaltige Zusammensetzung gemäß einem der Ansprüche 10 bis 12, wobei (G) während des Schritts des Mischens der Ölkomponenten ein polares Öl mit einem IOB-Wert von 0,1 bis 0,4 zugemischt wird.

14. Herstellungsverfahren für eine vesikelhaltige Zusammensetzung gemäß einem der Ansprüche 10 bis 13 zum Herstellen einer Zusammensetzung gemäß den Ansprüchen 5 bis 9.

15. Kosmetikprodukt, bestehend aus der vesikelhaltigen Zusammensetzung gemäß einem der Ansprüche 1 bis 9.

## Revendications

1. Composition contenant des vésicules, comprenant:
(A) une fragrance,
(B) une huile de silicone,
(C) un tensioactif de silicone,
(D) un ou plusieurs éléments choisis dans le groupe constitué par l'éthanol, le polypropylène glycol, le dipropylène glycol et le 1,3-butylène glycol et
(E) de l'eau,
dans laquelle le tensioactif de silicone (C) forme des vésicules et dans laquelle 95% en poids ou plus de la fragrance (A) et de l'huile de silicone (B) dans la composition sont présentes dans la membrane bicouche de vésicules.

2. Composition contenant des vésicules selon la revendication 1, dans laquelle le rapport de mélange de la fragrance (A) et de l'huile de silicone (B) est (A):(B) = 5:95 à 70:30 dans le rapport pondéral.

3. Composition contenant des vésicules selon la revendication 1 ou 2, en outre comprenant (F) un tensioactif soluble dans l'eau, de faible poids moléculaire.

4. Composition contenant des vésicules selon l'une des revendications 1 à 3, en outre comprenant (G) une huile polaire ayant une valeur IOB de 0,1 à 0,4.

5. Composition contenant des vésicules selon l'une des revendications 1 à 4, dans laquelle l'huile de silicone (B) est l'octaméthylcyclotétrasiloxane et/ou le décaméthylcyclotetrasiloxane.

6. Composition contenant des vésicules selon l'une des revendications 1 à 5, dans laquelle le tensioactif de silicone (C) est le silicone à modification polyoxyalkylène représenté par la formule générale (1) suivante, 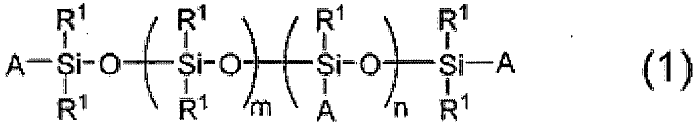 dans la formule (1) R¹ représente un atome d'hydrogène ou un groupe alkyle ayant 1 à 6 atomes de carbone pour A, au moins un des As étant un groupe représenté par la formule -(CH₂)ₐ-(C₂H₄O)_{b}-(C₃H₆O)_{c}-R² et les autres As étant un(des) atome(s) d'hydrogène ou un(des) groupe(s) alkyle ayant 1 à 6 atomes de carbone, le nombre m étant un nombre entier compris entre 1 et 200 et n étant un nombre entier compris entre 0 et 50; et dans la formule -(CH₂)ₐ-(C₂H₄O)_{b}-(C₃H₆O)_{c}-R², R² représente un atome d'hydrogène ou un groupe alkyle ayant 1 à 6 atomes de carbone, a étant un nombre entier compris entre 1 et 6, b étant un nombre entier compris entre 0 et 50, c étant un nombre entier compris entre 0 et 50 et b + c étant au moins 5 ou plus.

7. Composition contenant des vésicules selon l'une des revendications 3 à 6, dans laquelle le tensioactif soluble dans l'eau, de faible poids moléculaire (F) est un tensioactif ayant un poids moléculaire inférieur à 2000.

8. Composition contenant des vésicules selon l'une des revendications 1 à 7, dans laquelle le composant (D) est un ou plusieurs d'éléments choisis dans le groupe constitué par le propylène glycol, le dipropylène glycol et le 1,3-butylène glycol.

9. Composition contenant des vésicules selon l'une des revendications 4 à 8, dans laquelle le composant (D) est un ou plusieurs d'éléments choisis dans le groupe constitué par le tri (2-éthylhexanonate) de glycéryle, le isooctanoate de cétyle, le tétra (2-éthylhexanoate) de pentaérythritol et le isononanoate d'isononyle.

10. Procédé de fabrication d'une composition contenant des vésicules, le procédé comprenant:
une étape de mélange des composants d'huile, dans laquelle sont mélangés des composants huileux contenant (A) une fragrance et (B) une huile de silicone, et
une étape de formation des vésicules, dans laquelle le mélange de composants d'huile obtenu dans l'étape décrit ci-dessus est mélangé avec (C) un tensioactif de silicone et (D) un ou plusieurs éléments choisis dans le groupe constitué par l'éthanol, le polypropylène glycol, le dipropylène glycol et le 1,3-butylène glycol, et mélangé ensuite avec la formulation aqueuse contentant de l'eau pour former des vésicules du tensioactif de silicone (C), dans laquelle 95% en poids ou plus de la fragrance (A) et de l'huile de silicone (B) sont présentes dans la membrane bicouche de vésicules dans la composition obtenue.

11. Procédé de fabrication d'une composition contenant des vésicules selon la revendication 10, dans lequel le rapport de mélange de (A) la fragrance et de (B) l'huile de silicone est (A):(B) = 5:95 à 70:30 dans le rapport pondéral.

12. Procédé de fabrication d'une composition contenant des vésicules selon la revendication 10 ou 11, dans lequel (F) un tensioactif soluble dans l'eau, de faible poids moléculaire est ensuite ajouté et mélangé après l'étape de formation des vésicules.

13. Procédé de fabrication d'une composition contenant des vésicules selon l'une des revendications 10 à 12, dans lequel (G) une huile polaire ayant une valeur IOB de 0,1 à 0,4 est mélangé ensemble durant l'étape de mélange du composant d'huile.

14. Procédé de fabrication d'une composition contenant des vésicules selon l'une des revendications 10 à 13, pour produire une composition selon les revendications 5 à 9.

15. Produit cosmétique constitué par la composition contenant des vésicules selon l'une des revendications 1 à 9.
